# EUROPEAN PATENT APPLICATION

(11) **EP 1 548 031 A1**
(43) Date of publication of application: **29.06.2005**
(21) Application number: 03029541.4
(22) Date of filing: 22.12.2003
(51) Int. Cl.: C07K 14/505

(54) **Nature-identical erythropoietin**

(71) Applicant: Dubai Genetics FZ-LLC, Dubai Internet City, Dubai (AE)
(72) Inventor: Meijer, Hans, Dr., Dubai (AE); Al Ulama, Hafsa, Dubai (AE)
(74) Representative: Schrell, Andreas, Dr.

(57) **Abstract**

The present invention relates to Erythropoietin, (i.e. Haemopoietin, Haematopoietin, or erythropoietic stimulating factor) (EPO) having glycoform profiles or a glycoform close or identical to naturall occuring EPO (nEPO) as well as processes and means for the production thereof. The present invention also concerns usage of the EPO obtained according to the invention in connection with prophylactic or therapeutic treatment.

## Description

The present invention relates to Erythropoietin, (i.e. Haemopoietin, Haematopoietin, or erythropoietic stimulating factor) (EPO) having glycoform profiles or a glycoform close or identical to naturally occuring EPO, (nEPO) as well as processes and means for the production thereof. The present invention also concerns usage of the EPO obtained according to the invention in connection with prophylactic or therapeutic treatment use.

Naturally occurring Erythropoietin is a glycoprotein which is synthesised mainly in the kidney. Erythropoietin promotes the maturation of erythroid progenitor cells into erythrocytes, stimulates erythropoiesis through actions on erythroid progenitor cells, and is essential in regulating levels of red blood cells in the circulation. Conditions marked by low levels of tissue oxygen are related with increased production of EPO, which then upregulates erythropoiesis. An acute or progressive loss of kidney function, e.g. in chronic renal failure (CRF), most typically results in a decreased production of EPO concomitant with a reduction in red blood cells, decreased hematocrit, and anaemia.

As EPO is essential in red blood cell formation, the hormone is widely used in the treatment of blood disorders characterised by low or defective red blood cell production. At present, EPO is applied clinically in the treatment of anaemia in CRF patients (e.g. Eschbach, J.W. et al. 1987, 1988, 1989). EPO is further used in treatment of Acquired Immune Deficiency Syndrome (AIDS) and in cancer patients undergoing chemotherapy.

Until recently, the availability of EPO has been very limited. The protein is present, for example, in human urine. However, excreted levels are basically too low to make this a practical source of EPO for therapeutic use. Patients suffering from a plastic anaemia exhibit elevated levels of urinary EPO relative to healthy individuals, but limited availability of such patients also make such source impractical. The purification of human urinary EPO (hEPO) was described by Miake et al. (J. Biol. Chem. (1977) 252:5558).

The identification, cloning and expression of genes encoding EPO were described in EP 0 148 605 B1, the disclosure of which is incorporated herein by reference. A method for purification of recombinant EPO from mammalian cells containing recombinant Erythropoietin plasmids e.g. is described therein as well. Human EPO was the first haematopoietic growth factor to be cloned. At present, recombinant human EPO (rhEPO) is available as a drug in quantities suitable for therapeutic applications, in particular the clinical treatment of anaemia, especially anaemia caused by renal failure. In sports rhEPO has been used among some athletes for several years.

Active human EPO consists of a single 165 amino acid polypeptide chain with three N-glycosylation sites at asparagin residues located at positions, 24, 38 and 83, respectively, and one O-glycosylation site at a serine residue at position 126. The average carbohydrate content of the glycoprotein is approximately 40%.

The oligosaccharide chains have been shown to be modified with terminal sialic acid residues with N-linked chains typically having up to 4 sialic acids per chain and O-linked chains having up to two sialic acids. An EPO polypeptide may therefore accommodate up to a total of 14 sialic acids. Removal or modification of the glycan chains results in altered in vivo and in vitro activity. The number of sialic acid residues and the branching pattern of the N-linked oligosaccharides modify the pharmacodynamics, speed of catabolism, and biologic activity of EPO. So called EPO isoforms are described in EP 0 428 267 B1 and EP 0 668 351 A1.

Various studies have shown that alterations of EPO glycan structure or carbohydrate chains, respectively, can affect biological activity and/or pharmacokinetics of a molecule. The removal of one or more N-linked or O-linked oligosaccharide chains, for example, sharply reduces in vitro activity of the altered EPO (Dube et al., J. Biol. Chem. (1988) 263:17516). It was also found that a stepwise increase in sialic acid content per EPO molecule gave a corresponding stepwise increase in in vivo biological activity (Egrie et. al. Glycoconjugate J. (1993) 10:263). EPO isoforms having higher sialic acid content exhibited a longer serum half-life but showed a decreased affinity for the EPO receptor, suggesting that serum half-life is an important determinant of EPO in vivo biological activity. EPO glycosylation analogues having at least one additional carbohydrate chain have been determined to have a longer circulating half-life compared to recombinant human EPO.

Further attempts have been made to enhance the biological half-life of EPO. In one approach, the amino acid sequence has been modified to provide sites for additional glycosylation; more highly glycosylated forms exhibit this desirable property, as described in US 5,856,298. Yet another approach involves linking two EPO molecules together, as described in US 5,747,446. In another approach EPO is coupled at the O-terminus to the carboxy terminal portion (CTP) of the β-subunit of human chorionic gonadotropin, where the extended protein is recombinantly produced and secreted from CHO cells, as described in WO 03/394858.

In general, oligosaccharide units or glycans of glycoproteins contribute to the folding of nascent polypeptide chains, e.g. in the endoplasmatic reticulum, and serve to protect the protein moieties from the action of proteases, and serve to modulate biologic activities of a glycoprotein. In contrast to the synthesis of the polypeptide chain of a glycoprotein, which is genetically regulated, the oligosaccharide units or glycans are attached and processed by a series of enzymes reactions, the enzymes and enzyme compositions mainly being specific to a particular cell type or tissue. A glycoprotein thus generally appears as a mixture of different glycoforms resulting from varying enzymatic activity. Glycoform populations have been shown to be cell specific, tissue specific, species specific as well as polypeptide specific and site specific. Thus, each glycoprotein has a reproducible and characteristic glycosylation profile or glycosylation pattern.

The glycosylation profile or glycosylation pattern as well as the oligosaccharide structures of recombinant proteins also appear to be dependent on expression methods and culture conditions. Mammalian cell lines such as CHO or BHK cells are common hosts for the production of recombinant human Erythropoietin intended for therapeutic use. Three pharmaceuticals of rhEPO are available for clinical use. They are classified as EPO alpha, EPO beta, and EPO omega according to the manufacturing method. EPO alpha and beta are both produced in CHO cells, whereas EPO omega is produced in BHK cells.

It has been shown that EPO obtained from sera of anaemic patients have an apparent molecular weight slightly smaller than that of recombinant human EPO. Two dimensional gel electrophoresis reveal several different glycoforms and confirm the heterogeneity of circulating human EPO present in the human body (Skibeli V. et al. Blood (2001) 98(13): 3626-3634). Charge analysis demonstrated that human serum EPO contained only mono-, di- and tri-acidic oligosaccharides, but lacked the tetra-acidic structures present in the glycans from recombinant human EPO. The acidity of the oligosaccharide structures was caused by sialic acids (Skibeli V. et al. Blood (2001) 98(13): 3626-3634). The sugar profiles of human serum EPO, describing both neutral and charged sugar, appear significantly different from the profiles of recombinant human EPO; there exist discrepancies between human serum EPO and recombinant human EPO in respect to the glycan structures.

It is desirable to have a compound available with greater potency than the recombinant human EPO. An advantage to such a compound would be that it could be administered in some patients, or at least in one particular patient less frequently and/or at a lower dose. It is also desirable to have a compound available which represents a glycoform profile which is close or identical to the profile of EPO naturally occurring and/or produced in an animal or human body. An advantage to such a compound would be to prevent unwanted side effects connected with recombinant or foreign EPO or to prevent the detection of its presence in the body or in blood samples, in particular of some patients, or at least of one particular patient. It is also desirable to have a compound available wherein the negative side effects of its application, for example, red cell aplasia, death of athletes taking EPO, are minimised, in particular in some patients, or at least in one particular patient.

It is also desirable to have a more potent therapeutic for the treatment of anaemia available which, for example, will permit a less frequent dosing schedule. It is also desirable to have a compound available which will increase and maintain hematocrit at levels which are at least comparable to that of currently available recombinant human EPO when administered at lower dose. It is also desirable to have a compound available which is at least as well tolerated as recombinant human EPO, more preferably is better tolerated in some patients, or at least in one particular patient.

In particular, it is desirable to have a compound available which display pharmacokinetic properties which are similar or even improved in some patients, or at least in one particular patient and/or under at least one particular condition to the current pharmaceutical products and formulations of recombinant human EPO in respect to absorption, serum half-life and serum concentration levels. It is also desirable to have a compound available which presents less intense discomfort or no discomfort at all to the human or animal patient upon administration and/or exhibit much shorter duration of discomfort or no discomfort at the injection side. It is further desirable to have a molecule available which elicits no or a diminished immune response in comparison to currently available EPO upon administration in the human or animal body.

Accordingly, the problem underlying the present invention essentially is providing a novel production system for EPO and a novel EPO which most closely resembles the glycoform profile of naturally occurring EPO produced and present in an animal or human body, or single glycoforms or subpopulations of glycoforms thereof, which in particular allows for a very specific and even patient-specific treatment of a patient with an EPO being the same or essentially the same or bringing about the same effects as the EPO naturally produced in or being present in said patient, while at the same time having an availability which is comparable to or even higher than the availability of known recombinant EPO isolated from cell cultures or of EPO gained from urine, i.e., can be produced more effectively, cheaper, and more easily than known before.

The technical problem is solved by the provision of a process for the preparation of an Erythropoietin (EPO) from a cell or tissue in an in vitro system, comprising the steps of (a) providing
(i) at least one first cell or tissue, capable of inducing EPO production in a second cell or tissue, and
(ii) at least one second cell or tissue capable of producing EPO; (b) culturing the first cell or tissue (i) and the second cell or tissue (ii) in an in vitro system under conditions and for a time suitable to express, produce and secrete EPO into the culture medium; and (c) isolating the EPO produced from the culture medium. Preferably, the EPO is a natural or modified EPO.

Within the context of the present invention the terms "Erythropoietin" and "EPO" relate to a heterogenic population of different glycoforms of Erythropoietin glycoproteins, a special subpopulation of selected glycoforms of that glycoprotein, one individual EPO glycoform as well as mixtures of at least two glycoforms.

Within the context of the present invention the term "natural EPO" refers to EPO with a glycoform profile essentially identical or identical to "circulating" human EPO present in the human body or with a glycoform profile essentially identical or identical to EPO present in an animal body, in particular horse or birds of prey, more particular circulating equine EPO or avian EPO. The term "natural EPO" also refers to EPO with a glycoform profile essentially identical or identical to EPO naturally present in the human or animal body being in a certain physiological condition including starving, fasting, dehydration, physical exercise, altitude acclimation, anaemia, shock, coma, exanimation and sleep.

Within the context of the present invention the term "modified EPO" refers to derivatives, mutants, and variants of EPO, including truncated and fused EPO forms and EPO conjugates with further molecules. The term "modified EPO" also refers to subpopulations of EPO glycoforms, a single EPO variant or glycoform, and mixtures thereof.

A further embodiment of the present invention is EPO, in particular natural EPO, produced by the process according to the invention.

The present invention most advantageously provides EPO as a compound with greater potency than the recombinant human EPO, in particular for some patients and at least for one or more specific patients. The EPO according to the invention can advantageously be administered less frequently and/or at a lower dose, in particular for some patients and at least for one or more specific patients. The EPO according to the invention most advantageously represents a glycoform profile which is close or identical to the profile of EPO naturally occurring and/or produced in an animal or human body at a particular stage and/or condition, thus preventing unwanted side effects connected with recombinant or foreign EPO, in particular for some patients and at least for one or more specific patients.

Moreover, the therapeutically very useful EPO according to the invention prevents the detection of its presence in the body or in blood samples, in particular for some patients and at least for one or more specific patients, thereby avoiding any undesired interference with conventional doping controls.

The EPO according to the invention is a more potent therapeutic for the treatment of anaemia available which, for example, permits a less frequent dosing schedule. The EPO according to the invention, when administered at lower dose, increases and maintains hematocrit at levels which are at least comparable to that of currently available recombinant human EPO. The EPO according to the invention is at least as well tolerated as recombinant human EPO, and is better tolerated in some patients and at least in one or more specific patients.

The EPO according to the invention displays pharmacokinetic properties which are similar and/or improved over currently available pharmaceutical products and formulations of recombinant human EPO, in particular in respect to absorption, serum half-life, and serum concentration levels, in particular in some patients and at least in one or more specific patients. The EPO according to the invention further presents less intense discomfort or no discomfort at all to the human or animal patient upon administration and/or exhibits much shorter duration of discomfort or no discomfort at all at the injection site, in particular in some patients and at least in one or more specific patients.

The EPO according to the invention further elicits a diminished immune response in comparison to currently available EPO upon administration in the human or animal body; in particular in some patients and at least in one or more specific patients. In some patients the EPO according to the invention does not elicit any immune response at all.

In a preferred embodiment the first cell or tissue (i) is stimulated to induce the production of EPO in the second cell or tissue (ii). Preferably, the induction of the production of EPO in the second cell or tissue (ii) is mediated or mainly mediated by a soluble or diffusible factor released by the first cell or tissue (i).

According to the present invention the first cell or tissue is capable of producing EPO and/or capable of producing at least one diffusible signal stimulating and/or conferring the ability to other cells different from that cell to produce EPO. In one primary aspect of the present invention a first cell or tissue is inducible to express, produce and/or secrete at least one soluble factor and is capable of inducing or stimulating in second cell or tissue the expression, production and/or secretion of EPO into a medium, the first cell or tissue is also referred to as "EPO inducing". According to the present invention, the second cell or tissue is capable of expressing, producing and/or secreting EPO into a medium upon stimulation by at least one soluble factor expressed, produced and/or secreted by the first cell or tissue, the second cell or tissue is also referred to as "EPO producing". Preferably, the first cell or tissue is induced by physical or chemical stimulation, in particular by low oxygen (O₂) partial pressure, by electrical stimulation and/or chemical agents.

According to the invention cells or tissue are preferably selected as a first cell or tissue according to their ability to produce, upon stimulation, a high amount EPO stimulating signal and/or to confer high rate of EPO to the EPO producing second cell or tissue. According to the invention cells or tissue are preferably selected as the EPO producing second cell or tissue according to their ability to produce a high amount of EPO, preferably independent or mostly independent of culture conditions. Alternatively or in combination the EPO producing second cell or tissue are preferably cultured under conditions bringing about a high production rate of EPO. Of course, these conditions may vary for each individual cell type selected as the EPO producing second cell or tissue, but may be chosen according to standard proceedings.

According to the present invention the at least one EPO inducing first cell or tissue preferably is a mammalian cell particularly derived from renal tissue including endothelial and epithelial cells, in particular tubule cells, renal progenitor cells as well as cells which and exhibit at least one characteristic of a renal cell, in particular, the capability of producing and secreting EPO and/or at least one soluble signal mediating or stimulating the production and secretion of EPO from cells. Preferably, the cells are derived from kidney, liver, blood including lymphocytes and erythrocytes, or bone marrow, or from progenitor cells thereof. In another preferred embodiment the at least one EPO inducing first cell or tissue is an omnipotent stem cell or a pluripotent embryonic stem cell, preferably with at least one characteristic of a renal cell, in particular the ability to express, produce and secrete EPO. In yet another preferred embodiment the cell is a haematopoietic cell or derived from a progenitor cell thereof.

According to another preferred embodiment the first cell or tissue (i) and the second cell or tissue (ii) are selected from the same cell type, wherein the first cell or tissue (i) originates from a first host and/or first species and the second cell or tissue (ii) comprises or consists of cells originating from a second host and/or second species, and wherein the first host and/or first species is different from the second host and/or second species.

According to yet another preferred embodiment the first cell or tissue (i) is selected from a first cell type and the second cell or tissue (ii) comprises consist of or is selected from a second cell type, wherein the first cell type is different from the second cell type. In another preferred embodiment the second cell or tissue (ii) is of one cell type or of different cell types.

In preferred embodiments of the present invention the first cell or tissue (i) and/or the second cell or tissue (ii) are selected from the group consisting of cells, in particular primary cells or cultured primary cells, derived from kidney, liver, blood cells including lymphocytes, and erythrocytes, bone marrow and/or haematopoietic cells of the human or animal body and/or progenitor cells thereof, or immortalised mammalian cell lines including CHO, BHK, LLC-PK1, COS and human cell lines, and mixtures and/or co-cultures of at least two cell types, cell lines or tissues. In preferred embodiments the first cell (i) and/or the second cell or tissue (ii) originate from or are taken from a non-immortalised, non-modified cell culture or tissue, preferably primary cells. Preferably, the first cell or tissue (i) and/or the second cell or tissue (ii) originate from or are taken from human, horse, dog, camel mouse, rat, rabbit, bird, and simian.

Most preferably, the first cell or tissue (i) and/or the second cell or tissue (ii) is preferably chosen an "autologous cell or tissue" and is derived from the same body receiving the EPO produced by the cell or tissue.

In another preferred embodiment the first cell or tissue and/or the second cell or tissue is derived from organs taken from an animal or human body as an organ donor and/or in connection with therapeutic surgery, for example, removal of an organ or parts of an organ in connection with cancer therapy, in particular from kidney. Preferably, the in vitro system is used according to the invention to produce autologous EPO by autologous cells derived from organs of the same donor which will receive the EPO produced.

Alternatively, the cell is derived from cross-species tissue, e.g. from mouse, rat, rabbit, horse, bird, cat, dog, camel, simian or human. Preferably, the cell is derived from cross-species tissue, e.g. from mouse, rat, rabbit, horse, bird, cat, dog, camel, simian or human.

According to the invention cells may form three-dimensional aggregates. Preferably, the in vitro system is an artificial organ or an organotypic culture of such cells, in particular of kidney, liver or bone marrow.

In preferred embodiments either the first cell or tissue (i) or the second cell or tissue (ii) or both comprise at least one recombinant cell, preferably consist of recombinant cells. Preferably, the cell or tissue has been transformed with at least one recombinant nucleic acid molecule encoding EPO or derivates thereof.

In a preferred embodiment the recombinant cell is transformed with at least one recombinant nucleic acid molecule encoding EPO or derivates thereof. Preferably, the recombinant nucleic acid molecule codes for EPO with a glycoform profile typical for human, horse, bird, dog, or camel, respectively.

In a more preferred embodiment the nucleic acid sequence encoding the EPO is under control of at least one promoter and/or expression control element. Preferably, the expression control element is an oxygen responsive element.

In one preferred embodiment of the present invention the culturing of the first cell or tissue (i) and the second cell or tissue (ii) takes place in a shared cell culture compartment of the in vitro system. Preferably, first cell or tissue and second cell or tissue are co-cultured. According to a preferred embodiment the first cell or tissue (i) is identical to the second cell or tissue (ii).

In another preferred embodiment the culturing of the first cell or tissue (i) and the second cell or tissue (ii) takes place in at least two separate cell culture compartments of the in vitro system, wherein in a first compartment the first cell or tissue (i) is cultured and the second cell or tissue (ii) is cultured in at least one other compartment.

The present invention further includes a process to produce EPO in high purity and high quantity, a subpopulation of EPO glycoforms, an individual EPO glycoform, or a mixture of at least two EPO glycoforms, comprising the steps of (a) to (c) and (d) at least one further purification or isolation step. Preferably, the isolation step (c) and the purification or isolation step (d) are selected from reversed phase HPLC, HPLC, immunoaffinity chromatography, immunoaffinity-binding on particles including magnetic beads, cation and anion exchange chromatography, hydrophobicity chromatography, hydroxylapatit chromatography, dye affinity chromatography, lectin matrix purification, dihydroxybromyl matrix purification, gel filtration, salting out, precipitation with ammonium sulfate, isoelectric focussing, and a combination thereof. In a preferred embodiment the purification or isolation step (d) is a combination of affinity chromatography, anion exchange chromatography, and gel filtration. In a preferred embodiment the isolation step (c) includes immunoaffinity-binding on particles including magnetic beads, in particular, magnetic polystyrene beads coated with a protein A affinity-purified rabbit antibody against CHO cell-derived rhEPO. Preferably, the isolation step (c) further includes immunomagnetic purification with hEPO-specific magnetic beads. In a preferred embodiment the further purification or isolation step (d) is a combination of affinity chromatography, anion exchange chromatography, and gel filtration.

The present invention further includes EPO producible or produced by the process according to the invention. In a preferred embodiment the EPO comprises a subpopulation of glycoforms or variants or consists thereof. In another preferred embodiment the EPO comprises one individual glycoform or EPO variant or consists thereof.

Preferably, the EPO according to the invention is substantially free or free of human or animal blood products such as serum albumin including human serum albumin, equine serum albumin, and avian serum albumin.

In preferred embodiments the EPO according to the invention is human EPO derived from human cells or tissue, or equine EPO, canine EPO, and avian EPO, derived from equine, canine, or avian cells or tissue, respectively. In a further preferred embodiment the EPO according to the invention is recombinant EPO derived from recombinant cells, co-cultures, or tissue thereof, in particular immortalised cell lines, with at least one recombinant gen construct in connection with EPO production.

In a preferred embodiment the EPO according to the invention is producible or produced by further processing according to any known method forming a conjugate of EPO, wherein EPO is covalently linked to polyethylene glycol or equivalents.

In a preferred embodiment the EPO according to the invention is a hyperglycosylated analogue of the EPO produced or producible according to the invention.

In a preferred embodiment the EPO according to the invention is producible or produced by further processing EPO according to any known method such as to modify to reduce immunogenicity of EPO and/or to diminish or prevent adverse effects of an immune response of the human or animal body upon administration of EPO.

Another embodiment of the present invention is a pharmaceutical composition comprising EPO according to the invention together with one or more pharmaceutically acceptable excipients. In a preferred embodiment the pharmaceutically acceptable excipient is selected from the group consisting of inorganic salts, pH buffers, amino acids, polyols, diluents, solvents, carriers, stabilisers, solubilisers, emulsifiers, preservatives, non-ionic detergents, surfactants, tonicity agents, anti-oxidants, and adjuvants. Preferably, the excipient is selected from the group consisting of sodium chloride, glucose, citrate, acetate and phosphate buffered systems, urea, human, equine or bovine serum albumin, lecithin, polyethylene glycol, mannitol, sorbitol, benzyl alcohol, ethanol, parabens, phenols, cresol, polysorbate 80, polysorbate 20, pluronic F68, glycine, methionine, vitamin C, vitamin A, and vitamin E.

Another embodiment of the present invention is a pharmaceutical composition comprising EPO according to the invention together with one or more further compounds. In a preferred embodiment the compound is selected from the group consisting of amino acids, polyols, antioxidants, vitamins, trace elements, iron, anti-tumor agents, antineoplastic agents, antiproliferative agents, cytostatica, anti-apoptotic agents, toxines, enyzmes, diagnostic imaging or contrast agents, dyes, antibacterial agents, antifungal agents, antiviral agents, cytostatics, immunosuppressive agents, analgesic agents, hormones, anti-inflammatory agents, and haematopoietic agents.

In a preferred embodiment the pharmaceutical composition is an aqueous formulation, a lyophilised or a spray dried formulation.

The present invention also concerns methods of treatment and the use of EPO according to the invention for therapeutic and/or prophylactic treatment of diseases curable with EPO. In particular methods of treatment and the use of EPO for the therapeutic and/or prophylactic treatment of: (a) diseases in connection with anaemia, including nephrogenic anaemia such as CRF related anaemia; (b) anaemia secondary to treatment with anti-viral drugs, anti-proliferative drugs, anti tumor agents, antineoplastic agents, and immunosuppressive agents; (c) anaemia secondary to treatment of HIV infection, (d) anaemia secondary to chemotherapeutic or radiation regimens including chemotherapy and radiation therapy in connection with cancer such as myelosuppressive therapy, in particular with anti-tumor agents including TNF and cisplatin; (e) anaemia associated with rheumatoid arthritis, prematury, excessive blood loss, myelofibrosis, sickle cell anaemia, bone marrow transplantation, thermal injury, β-thalassemia, and Acosta's disease; and (f) diseases in connection with acute or chronic ischemic injury of the myocardium, skeletal muscle cells or renal cells.

The present invention also concerns methods of treatment and the use of EPO according to the invention for improving peripheral oxygenation, improving physical performance, facilitating presurgical autologous blood donation, and/or maintaining or increasing hematocrit values in an animal or human body.

The present invention also concerns methods of treatment and the use of EPO according to the invention for preventing and treating ischemic acute renal failure, cardiac failure, congestive heart failure, endothelial injury such as inflammation, diseases of the central nervous system, diseases of the peripheral nervous system, and harmful cell apoptosis or necrosis such as in renal tubular cells myocardial cells, muscle cells, liver cells, bone marrow, and in central nervous tissue such neuronal death in an animal or human body.

The present invention also concerns methods of treatment and the use of EPO according to the invention for inducing, stimulating and/or supporting the formation of new blood vessels, neovascularisation, angiogenesis, vasoproliferative processes, neuroprotection, mitosis, proliferation, cell motility, and wound healing in an animal or human body.

The present invention also concerns methods of treatment and the use of EPO according to the invention as a hormone.

In a preferred embodiment the method of use and the use of EPO comprises the step of administering EPO in a therapeutically or prophylactically effective dose, in particular in form of a pharmaceutical composition according to the invention. Preferably, EPO is administered in a dose from 10 IU to 100 000 IU, preferably from 500 IU to 2000 IU. In a preferred variant EPO is administered in a dose of 0.5 IU to 2000 IU per kg body weight.

The present invention also concerns methods of use and the use of EPO, wherein EPO is produced by at least one autologous cell or autologous tissue from an animal or human body, in particular cultured autologous cells derived from the same body, and EPO is administered to the same body.

The present invention also concerns the use of EPO according to the invention for the preparation of a medicament or a pharmaceutical preparation for the treatment or use according to the invention.

In another aspect of the invention an in vitro cell culture or in vitro tissue culture system usable within the context of the process for the production of EPO according to the invention is concerned. Preferably, the in vitro cell culture or in vitro tissue culture system is used to produce EPO according to the invention.

In a preferred embodiment the in vitro system is operable or operated in a continuous mode. In another preferred embodiment the in vitro system is operable or operated in a continuous mode. In another preferred embodiment the in vitro system is operable or operated in a semi-continuous mode. In yet another preferred embodiment the in vitro system is operable or operated in a batch-wise operation mode.

In a preferred embodiment the in vitro system is a cell culture system for suspension culture. In another preferred embodiment the in vitro system is a cell culture system for supported culture with the cell or tissue culture being attached to at least on support, matrix or scaffold.

Preferably, the in vitro system is operated in the context of the invention for autologous EPO transfer, wherein the in vitro system comprises autologous cells and/or tissue or consisting solely thereof, i.e., cell or tissue come from the individual patient to be treated. In another preferred embodiment the in vitro system is operated in the context of the invention for heterologous EPO transfer, wherein the in vitro system comprises mainly heterologous cells and/or tissue, i.e., cell or tissue originate from an individual belonging to the same species as the patient to be treated. In yet another preferred embodiment the in vitro system is operated in the context of the invention for cross-species EPO transfer, wherein the in vitro system comprises mainly cross-species cells and/or tissue, i.e., cell or tissue originate from an individual belonging to a species different to the species of the patient to be treated.

In a preferred embodiment the in vitro system comprises at least one support for first cells or tissues and second cells or tissues, one or more cell culture compartments and a culture medium.

In a more preferred embodiment the support of the in vitro system is connected or borders at at least one side to one or more cell culture compartments containing the cells or tissue which is suppliable with culture medium, preferably liquid culture medium. Preferably, the cell or tissue is contained in at least two cell culture compartments.

In a preferred embodiment the at least two cell culture compartments are separated from each other by a barrier, which inhibits cell migration from one compartment to another compartment, but allows the migration or diffusion of molecules from at least one compartment to another compartment.

In a preferred embodiment the culture medium which preferably contains serum or is serum-free is supplied to the at least one cell culture compartment which preferably has at least one inflow and/or at least one outflow openings.

In a preferred embodiment the in vitro system includes one or more gas compartments preferably suppliable with a gas or gas mixture. Preferably, at least one gas compartment the gas compartment is connected with, is corresponding with or borders to at least one of said cell culture compartments, such that at least one gas diffuses across the connection or border between the gas compartment and the cell culture compartment. In another preferred variant the gas compartment is connected with, is corresponding with or borders to at least one culture medium supplied to at least one of said cell culture compartments.

In a preferred embodiment the at least one cell culture compartment contains culture medium different from culture medium present in another cell culture compartment. and/or a different partial pressure of at least one gas in comparison to another cell culture compartment.

In another preferred embodiment the partial pressure of at least one gas present in the culture medium of at least one cell culture compartment is different from the partial pressure of this gas in another cell culture compartment. Preferably, at least two cell culture compartments are supplied with at least one different gas or gas mixture.

In a preferred embodiment the culturing step (b) of the process according to the invention is performed under a condition of reduced partial pressure of oxygen prevailing in at least one cell culture compartment. Preferably, the condition of reduced partial pressure of oxygen is prevailing in at least one cell culture compartment for an interrupted period of time. In a preferred alternative the condition of reduced partial pressure of oxygen is prevailing in at least one cell culture compartment for a period of time sufficient to induce or increase the production and/or release of EPO. Preferably, in both alternatives, the partial pressure of oxygen is, at the same time, normal or increased in another compartment.

In a preferred embodiment the cells are cultured in monolayers. In another preferred embodiment the cells the in vitro system is an artificial organ or an organotypic culture. In a preferred embodiment the growth support is in form of a three dimensional matrix or scaffold, in particular for an organoid or organotypic culture. In preferred embodiments the support is in solid form, in particular fibrous or porous form including sponges, foams, porous fabrics, or in gel form. Preferably, the growth support comprises a microporous structure, in particular a filter mesh or filter paper.

In other preferred embodiments the growth support comprises or consists of collagen, alginate, cellulose, polyhydroxyalkanoate, proteoglycans, agarose, gelatin, hyaluronan, or derivatives thereof, as well as synthetic polymers including PTFE, vicryl-polydioxanon-copolymers, polyglycolic acid, polyalkylene glycol-aromatic polyester-copolymers, and PE, or a composite of different materials thereof.

In a preferred embodiment of the present invention at least one culture medium comprises one or more growth factor or cytokine selected from the group consisting of granulocyte-macrophage colony stimulating factor (GM-CSF), IL-3, granulocyte colony stimulating factor (G-CSF), transformin growth factor-β (TGF-β), platelet derived growth factor (PGF), insulin like growth factor (IGF), acidic fibroblast growth factor (aFGF), basic fibroblast growth factor (bFGF), epidermal growth factor (EGF), hepatocytic growth factor (HGF), keratocyte growth factor (KGF), and neural growth factor (NGF). Preferably, particular the culture medium comprises GM-CSF, IL-3, and/or G-CSF.

In a preferred embodiment of the present invention the in vitro cell culture system is a system as patented in US 6,329,195 B1, the disclosure content of which is fully incorporated herein by reference as one preferred embodiment. In particular the structure, principle and design of the disclosed system as depicted, for example, in figures 1 to 5 and as explained in claims 2 to 5 of said patent.

In particular, this in vitro system consists of at least two major separate parts, namely at least one, preferably two, preferably cylindrical, cell culture compartment, carrying at least one support for cells or a tissue to be cultured and being provided with at least one cell culture medium, and a, preferably cylindrical, gas compartment allowing for permanent supply of respiratory gases. In a more preferred embodiment the cell culture compartment comprises a specifically designed cylindrical piece and at least one growth support, the growth support being arranged within, preferably clamped into, the cylindrical piece of the cell culture compartment, preferably by a plastic mounting ring. The cell culture compartment further comprises walls which are supplied with at least one inlet and at least one outlet for cell culture medium, wherein at least one inlet and one outlet is arranged on both sides of the growth support which is arranged within the cell culture compartment. In a preferred embodiment these in- and outlets, also designated as medium channels, comprise semi-circular openings directed towards the inside of the cell culture compartment in such a way that the fluid compartments formed on both sides of the support are well mixed mainly due to turbular fluid movement.

In a preferred embodiment at least one of the cell culture compartments comprises at least one hole or opening, in particular two holes, forming a gas channel which is a connection to the at least one gas compartment bordering to each cell culture compartment. The gas compartment further comprises in- and outlets, in particular in forms of vertical and horizontal bores, for the in- and out-flowing gas phase.

In a preferred embodiment the cell culture compartment is provided in the form of a hollow cylinder or cup which is closed on the openings with a membrane which is gas permeable but impermeable to liquids such as water. In a preferred embodiment the gas permeable membrane is a PTFE membrane, preferably approximately 100 µm of thickness. Preferably, the membrane is fixed to a support grid, e.g. by means of a clamping ring. Preferably, the gas compartment is in intimate contact with the cell culture compartment in such a way that permanent diffusion and equilibration of the gases in the medium perfused through the cell culture compartment and the gas or gases present in the gas compartment is accomplished. In a preferred embodiment cell culture compartment and gas compartment are connected such that a closed volume is formed within the cell culture compartment limited by the walls of the cell culture compartment, the growth support carrying cells or tissue, and the gas permeable membrane of the gas compartment, the circular gas permeable membrane and the circular growth support being arranged preferably concentrically and parallel.

Although only preferred embodiments of the invention are specifically described above and in the following examples, the embodiments and examples are provided to more clearly illustrate the aspects of the invention and are not intended to limit the scope of the present invention. Further modifications and variations of the invention are possible within the scope of invention.

### Example 1: EPO production

In the following example an EPO according to the invention is produced by differentiated renal cells.

### Methods

Primary equine renal cells were cultured statically, the cell culture apparatus was seeded and the apparatus the cells were induced to produce EPO.

### a) Cell culture

Equine proximal tubular epithelial cells (PTECs) were isolated as follows. Equine renal cortical tissue was obtained from kidneys taken from euthanised healthy animals. Cortical specimens were cut into small cubes and passed through a series of mesh sieves of diminishing pore size. PTECs were collected on a 53 µm sieve and digested with collagenase (750 U/ml) at 37°C for 15 min. Tubular cells were isolated by centrifugation and grown in a 1:1 mixture of DMEM and Ham's F12 medium supplemented with 10%FCS, hydrocortisone (40 ng/ml), L-glutamine (2 mmol/l), benzyl penicillin (100 IU/ml), and streptomycin (100 µg/ml). The cells were incubated at 37°C in 5% CO₂ and 95% air. The cells were characterized microscopically and immuno-histochemically to be of proximal tubular origin. The cell were grown to confluence on Millicell HA filters (Millipore®) and transferred to an Epiflow chamber (Felder et al., Cell Physiol. Biochem. (2002) 12:153-162); US 6,329,195 B1) with a medium transfusion rate of 3 ml/hour and an air delivery rate of 10 ml/min.

### b) EPO induction

The oxygen concentration was decreased from 20% to 10% by an increase of the nitrogen concentration. CO2 concentration was kept constant at 5% for 20 min. The flow through was collected in fractions of 0.5 ml, which corresponded to 10 min.

### c) EPO ELISA

Concentration of EPO flow-through was determined by an ELISA assay (Stem ell Technologies Inc.), according to the manufacturer's instructions.

### Results

Reduction of the oxygen concentration induces production of EPO.

### Example 2: EPO structure

In the following example the difference in structure between three EPO forms, including EPO obtained according to example 1, is investigated.

### Methods

EPO proteins were purified and the structure of the molecules was investigated. 5 samples were analysed:
- sample 1:: NESP in culture medium, no purification
- sample 2:: NESP in culture medium, purification
- sample 3:: nEPO in culture medium, purification
- sample 4:: serum from a healthy donor

### a) Preparation of Erythropoietin-specific magnetic beads

Magnetic polystyrene beads (Dynal®) were coated with a protein A affinity-purified rabbit antibody against CHO cell-derived rhEPO (R&D Systems Inc.) according to the manufacturer's instructions.

Binding of all EPO to the antibody-coated beads was performed for 48 hours at ambient temperature.

### b) Immunomagnetic purification

To reduce unspecific binding, the samples were treated with polyethylene glycol, PEG 6000, 12.5% wt./vol. to precipitate immunoglobulines from the culture medium and were centrifuged at 3500 g for 20 min before incubation with the antibody coated magnetic beads. Human EPO-specific magnetic beads (130 mg) were incubated with 4 ml sample. Due to the capacity of the beads, approximately 6.5 pmol EPO was extracted and subjected to further analyses.

Subsequently, the beads were washed with phosphate buffered saline (PBS; Invitrogen) containing Tween-20 (1% vol/vol), then with 0.5 mol/l NaCl, and finally with 0.15 mol/I NaCl. Serum EPO was eluted from the beads by incubation with 1% sodium dodecyl sulfate (SDS) in PBS at ambient temperature under vigorous shaking for 30 min. SDS was removed by dialysis against PBS and subsequently human serum EPO was concentrated by ultrafiltration in Centricon-30 (Millipore).

### c) SDS-PAGE and immunoblotting

Subsequent to reducing SDS-polyacrylamide gel electrophoresis (PAGE) in 12% gels (Xcell SureLock; Invitrogen), the proteins were blotted onto poly-vinylidene difluoride membranes (0.2 mm; BioRad), in a semidry blotting apparatus (Xcell SureLock®; Invitrogen). After blotting, the membranes were blocked with 3% bovine serum albumin in PBS for 5 hours and were incubated with a biotinylated monoclonal anti-hEPO antibody and streptavidin-alkaline phosphatase (R&D).

### Results

No qualitative or obvious quantitative differences could be detected between sample 1 and sample 2 indicating that the purification does not affect the EPO structure. Sample 3 (nhEPO) and sample 4 (serum EPO) migrate faster than sample 1 and sample 2 (NESP). No qualitative or obvious quantitative differences could be detected between sample 3 and 4.

### Example 3: EPO activity

The activity of NESP, nhEPO and serum EPO was compared. The specific EPO activity was determined by determining EPO concentration and activity.

### Methods

### a) EPO ELISA

Concentration of EPO was determined by an ELISA assay (StemCell Technologies Inc.), according to the manufacturer's instructions.

### b) Haematopoietic Colony Assay

The colony assays were set up by using human CD34 mononuclear cells isolated from healthy donors using the macs® system (Miltenyi Biotech). Approximately 1000 to 2000 cells were mixed with 1 ml of methylcellulose culture medium containing FBS (Invitrogen), 0.4% DMSO, a mixture of growth factors containing granulocyte-macrophage colony stimulating factor (GM-CSF), IL-3 and granulocyte colony stimulating factor (G-CSF) and test compound (NESP, nhEPO or serum EPO). After mixing, the suspension was plated in 35-mm gridded plates (Nunc) and incubated at 37°C in a humidity controlled CO₂ incubator. Erythroid colony-forming units (CFU) containing > 50 heemoglobinised cells, myeloid colonies (CFU-GM) containing > 50 cells and mixed colonies, containing both erythroid and myeloid cells were counted on day 14-16.

### Results

NESP has a lower activity than nhEPO and serum EPO. No significant difference was found between nhEPO and serum EPO.

### Example 4: Recombinant EPO

In the following the production of recombinant EPO in a renal cell culture system according to the invention is described.

### Methods

### a) Construction of EPO plasmid

For the following experiments EPO coding constructs based on genomic and cDNA sequences were prepared.

### i) Genomic construct

Into the genomic construct identified below 3 copies of the oxygen responsive element identified below as well were inserted into the first intron.

### ii) cDNA constructs

From the mRNA identified below a cDNA was prepared by conventional methods. 5' to the coding region of EPO in one construct the endogenous EPO promoter and in another construct the CMV promoter was cloned. In another set of two cDNA constructs the oxygen responsive element identified below was cloned between the endogenous or CMV promoter and the EPO cDNA sequence.

### iii) Materials used:

### 1) Homo sapiens EPO mRNA

LOCUS NM_000799 1342 bp mRNA linear PRI 06-OCT-2003 Accession NM_000799 (NM_000799.1 GI:4503588)

### 2) EPO genomic locus (Human erythropoietin gene, complete cds)

LOCUS HUMERPA 3602 bp DNA linear PRI 08-NOV-1994, Accession M11319 (M11319.1 GI:182197)

### 3) O₂ responsive element

Hinf I to Pvu II (227 bp) from huEPO promoter

### b) Transformation and cell culture

The constructs were transformed, primarily in a mammalian cell line including CHO, BHK, and COS, by conventional methods. The transformants were selected and diluted by conventional methods, and then plated in the above described in vitro system in either high or low oxygen pressure.

### c) EPO induction

The oxygen concentration was decreased from 20% to 10% by an increase of the nitrogen concentration. CO₂ concentration was kept constant at 5% for 20 min. The flow through was collected in fractions of 0.5 ml, which corresponded to 10 min.

### d) EPO ELISA

Concentration of EPO flow-through was determined by an ELISA assay (StemCell Technologies), according to the manufacturer's instructions.

### Results

The reduction of the oxygen concentration induces production of EPO.

## Claims

1. A process for the preparation of an erythropoietin (EPO) from a cell or tissue in an in vitro system, comprising the steps of:
(a) providing
(i) at least one first cell or tissue, capable of inducing EPO production in a second cell or tissue, and
(ii) at least one second cell or tissue capable of producing EPO;
(b) culturing the first cell or tissue (i) and the second cell or tissue (ii) in an in vitro system under conditions and for a time suitable to induce EPO production and to express, produce and secrete EPO into the culture medium; and
(c) isolating the EPO produced from the culture medium.

2. A process according to claim 1 wherein the EPO is a natural or modified EPO.

3. A process according to any one of the preceding claims, wherein the first cell or tissue (i) is stimulated to induce the production of EPO in the second cell or tissue (ii).

4. A process according to any one of the preceding claims, wherein the first cell or tissue (i) is stimulated by physical stimulation including electrical stimulation.

5. A process according to any one of the preceding claims, wherein the first cell or tissue (i) is stimulated by chemical stimulation including stimulation with at least one chemical compound.

6. A process according to any one of the preceding claims, wherein the first cell or tissue (i) is stimulated by reduced oxygen (O₂) partial pressure.

7. A process according to any one of the preceding claims, wherein the induction of the production of EPO in the second cell or tissue (ii) is mediated by a soluble or diffusible factor released by the first cell or tissue (i).

8. A process according to any one of the preceding claims, wherein the first cell or tissue (i) is stimulated to induce the production of EPO in the second cell or tissue (ii).

9. A process according to any one of the preceding claims, wherein the first cell or tissue (i) is identical to the second cell or tissue (ii).

10. A process according to any one of the preceding claims, wherein the first cell or tissue (i) and the second cell or tissue (ii) are selected from the same cell type, wherein the first cell or tissue (i) originates from a first host and/or first species and the second cell or tissue (ii) comprises or consists of cells originating from a second host and/or second species, and wherein the first host and/or first species is different from the second host and/or second species.

11. A process according to any one of the preceding claims, wherein the first cell or tissue (i) is selected from a first cell type and the second cell or tissue (ii) comprises consist of or is selected from a second cell type, wherein the first cell type is different from the second cell type.

12. A process according to any one of the preceding claims, wherein the second cell or tissue (ii) is of one cell type or of different cell types.

13. A process according to any one of the preceding claims, wherein the first cell or tissue (i) and/or the second cell or tissue (ii) are selected from the group consisting of organ cultures, primary cells or cultured primary cells, derived from kidney including kindney from an autologous donor, liver, blood cells including lymphocytes, and erythrocytes, bone marrow and/or haematopoietic cells of the human or animal body and/or progenitor cells thereof, immortalised mammalian cell lines including CHO, BHK, LLC-PK₁, COS, and mixtures and/or co-cultures of at least two cell types thereof.

14. A process according to any one of the preceding claims, wherein the first cell or tissue (i) and/or the second cell or tissue (ii) comprise at least one recombinant cell or consist thereof.

15. A process according to claim 13 wherein the recombinant cell is transformed with at least one recombinant nucleic acid molecule encoding EPO or derivates thereof.

16. A process according to claim 15 wherein the recombinant nucleic acid molecule codes for EPO with a glycoform profile typical for human, horse, bird, dog, or camel, respectively.

17. A process according to claim 15 or 16 wherein the nucleic acid sequence encoding the EPO is under control of at least one promoter and/or expression control element.

18. A process according to claim 17 wherein the expression control element is an oxygen responsive element.

19. A process according to any one of the preceding claims, wherein in the in vitro system the culturing the first cell or tissue (i) and the second cell or tissue (ii) takes place in a shared cell culture compartment.

20. A process according to any one of the preceding claims, wherein in the in vitro system the culturing of the first cell or tissue (i) and the second cell or tissue (ii) takes place in at least two separate cell culture compartments, wherein in a first compartment the first cell or tissue (i) is cultured and the second cell or tissue (ii) is cultured in at least one other compartment.

21. A process according to any one of the preceding claims, wherein the in vitro system comprises at least one support for first cells or tissue and/or second cells or tissue, one or more cell culture compartments and a culture medium.

22. A process according to any one of the preceding claims, wherein the support is connected or borders at least one side to the cell culture compartment.

23. A process according to any one of the preceding claims, wherein the cell culture compartment is suppliable with liquid culture medium.

24. A process according to any one of the preceding claims, wherein the culture medium contains serum or is serum-free.

25. A process according to any one of the preceding claims, wherein the cell culture compartments are separated from each other by a barrier, which inhibits cell migration from one compartment to another compartment, but allows the migration or diffusion of molecules from at least one compartment to another compartment.

26. A process according to any one of the preceding claims, wherein the in vitro system includes at least one gas compartment which is suppliable with a gas or gas mixture.

27. A process according to any one of the preceding claims, wherein the gas compartment is connected with, is corresponding with or borders to at least one of said cell culture compartments, such that at least one gas diffuses across the connection or border between the gas compartment and the cell culture compartment.

28. A process according to any one of the preceding claims, wherein the gas compartment is connected with, is corresponding with or borders to at least one culture medium supplied to at least one of said cell culture compartments.

29. A process according to any one of the preceding claims, wherein in at least one cell culture compartment a different culture medium and/or a different partial pressure of at least one gas is contained in comparison to another cell culture compartment.

30. A process according to any one of the preceding claims, wherein at least two cell culture compartments are supplied with different gas or gas mixtures.

31. A process according to any one of the preceding claims, wherein in step (b) the culturing is performed under a condition of reduced partial pressure of oxygen prevailing in at least one cell culture compartment.

32. A process according to claim 28, wherein the condition of reduced partial pressure of oxygen is prevailing in at least one cell culture compartment for an interrupted period of time.

33. A process according to claim 28, wherein the condition of reduced partial pressure of oxygen is prevailing in at least one cell culture compartment for a period of time sufficient to induce or increase the production and/or release of EPO.

34. A process according to claim 28, wherein the partial pressure of oxygen is normal or increased in another compartment.

35. A process according to any one of the preceding claims, wherein at least one culture medium comprises one or more growth factor or cytokine selected from the group consisting of granulocyte-macrophage colony stimulating factor (GM-CSF), IL-3, granulocyte colony stimulating factor (G-CSF), transformin growth factor-β (TGF-β), platelet derived growth factor (PGF), insulin like growth factor (IGF), acidic fibroblast growth factor (aFGF), basic fibroblast growth factor (bFGF), epidermal growth factor (EGF), hepatocytic growth factor (HGF), keratocyte growth factor (KGF), and neural growth factor (NGF), in particular GM-CSF, IL-3, and G-CSF.

36. A process according to any one of the preceding claims, wherein the cells are cultured in monolayers.

37. A process according to any one of the preceding claims, wherein the in vitro system is an artificial organ or an organotypic culture.

38. A process according to any one of the preceding claims, wherein the support is in form of a three-dimensional matrix or scaffold.

39. A process according to any one of the preceding claims, wherein the support comprises or consists of collagen, alginate, cellulose, polyhydroxyalkanoate, proteoglycans, agarose, gelatin, hyaluronan, or derivatives thereof, as well as synthetic polymers including PTFE, vicryl-polydioxanon-copolymers, polyglycolic acid, polyalkylene glycol-aromatic polyester-copolymers, and PE, or a composite of different materials thereof.

40. A process according to any one of the preceding claims, wherein the support is in solid form, in particular fibrous or porous form including sponges, foams, porous fabrics, or in gel form.

41. A process to produce EPO in high purity, a subpopulation of EPO glycoforms, an individual EPO glycoform, or a mixture of at least two EPO glycoforms, comprising the steps:
(a) to (c) according to any one of the preceding claims, and
(d) at least one further purification step selected from reversed phase HPLC, HPLC, immunoaffinity chromatography, immunoaffinity magnetic beads, cation and anion exchange chromatography, hydrophobicity chromatography, hydroxylapatit chromatography, dye affinity chromatography, lectin matrix purification, dihydroxybromyl matrix purification, gel filtration, salting out, precipitation with ammonium sulfate, isoelectric focussing, and a combination thereof.

42. EPO produced by the process according to any one of claims 1 to 39.

43. EPO according to claim 42 comprising or consisting of a subpopulation of glycoforms.

44. EPO according to claim 42 comprising or consisting of an individual glycoform.

45. EPO according to any one of claims 42 to 43 being substantially free of human or animal blood products such as serum albumin.

46. EPO according to any one of claims 42 to 45 being human EPO, equine EPO, canine EPO, avian EPO, or an recombinant EPO.

47. EPO according to any one of claims 42 to 46 produced by further processing EPO and forming a conjugate of EPO wherein EPO is covalently linked to polyethylene glycol.

48. EPO according to any one of claims 42 to 47 that has been further modified to reduce immunogenicity and/or to prevent adverse effects of an immune response upon administration.

49. Pharmaceutical composition comprising EPO according to any one of claims 42 to 48 and one or more pharmaceutically acceptable excipients and/or further compounds.

50. Pharmaceutical composition according to claim 49 wherein the pharmaceutically acceptable excipient is selected from the group consisting of inorganic salts, pH buffers, amino acids, polyols, diluents, solvents, carriers, stabilisers, solubilisers, emulsifiers, preservatives, non-ionic detergents, surfactants, tonicity agents, anti-oxidants, and adjuvants.

51. Pharmaceutical composition according to claim 50 wherein the pharmaceutically acceptable excipient is selected from the group consisting of sodium chloride, glucose, citrate, acetate and phosphate buffered systems, urea, human, equine or bovine serum albumin, lecithin, polyethylene glycol, mannitol, sorbitol, benzyl alcohol, ethanol, parabens, phenols, cresol, polysorbate 80, polysorbate 20, pluronic F68, glycine, methionine, vitamin C, vitamin A, vitamin E.

52. Pharmaceutical composition according to any one of claims 49 to 51 wherein the compound is selected from the group consisting of amino acids, polyols, antioxidants, vitamins, trace elements, iron, anti-tumor agents, antineoplastic agents, antiproliferative agents, cytostatica, anti-apoptotic agents, toxines, enyzmes, diagnostic imaging or contrast agents, dyes, antibacterial agents, antifungal agents, antiviral agents, cytostatics, immunosuppressive agents, analgesic agents, hormones, anti-inflammatory agents, and haematopoietic agents.

53. Pharmaceutical composition according to any one of claims 49 to 52 being an aqueous formulation, lyophilised, or spray dried.

54. Use of EPO according to any one of claims 42 to 48 for therapeutic and/or prophylactic treatment of diseases curable with EPO.

55. Use of EPO according to any one of claims 42 to 48 for therapeutic and/or prophylactic treatment of
(a) diseases in connection with anaemia, including nephrogenic anaemia such as CRF related anaemia;
(b) anaemia secondary to treatment with anti-viral drugs, anti-proliferative drugs, anti tumor agents, antineoplastic agents, and immunosuppressive agents;
(c) anaemia secondary to treatment of HIV infection,
(d) anaemia secondary to chemotherapeutic or radiation regimens including chemotherapy and radiation therapy in connection with cancer including myelosuppressive therapy;
(e) anaemia associated with rheumatoid arthritis, prematury, excessive blood loss, myelofibrosis, sickle cell anaemia, bone marrow transplantation, thermal injury, β-thalassemia, and Acosta's disease; and
(f) diseases in connection with acute or chronic ischemic injury of the myocardium, skeletal muscle cells or renal cells.

56. Use of EPO according to any one of claims 42 to 48 for improving peripheral oxygenation, improving physical performance, facilitating presurgical autologous blood donation, and/or maintaining or increasing hematocrit values in an animal or human body.

57. Use of EPO according to any one of claims 42 to 48 for preventing and treating ischemic acute renal failure, cardiac failure, congestive heart failure, endothelial injury such as inflammation, diseases of the central nervous system, diseases of the peripheral nervous system, and harmful cell apoptosis or necrosis such as in renal tubular cells myocardial cells, muscle cells, liver cells, bone marrow, and in central nervous tissue such neuronal death in an animal or human body.

58. Use of EPO according to any one of claims 42 to 48 for inducing, stimulating and/or supporting the formation of new blood vessels, neovascularisation, angiogenesis, vasoproliferative processes, neuroprotection, mitosis, proliferation, cell motility, and wound healing in an animal or human body.

59. Use of EPO according to any one of claims 42 to 48 as a hormone.

60. Use according to any one of claims 54 to 59, wherein EPO is administered in a dose from 10 IU to 100 000 IU, preferably from 500 IU to 2000 IU.

61. Use according to any one of claims 54 to 59, wherein EPO is administered in a dose of 0.5 IU to 2000 IU per kg body weight.

62. Use according to any one of claims 54 to 61 comprising the step of administering EPO in a therapeutically or prophylactically effective dose, in particular in form of a pharmaceutical composition according to any one of claims 42 to 48.

63. Use according to any one of claims 52 to 62, wherein EPO is produced by at least one autologous cell or autologous tissue from an animal or human body, in particular cultured renal cells, and EPO is administered to the same animal or human body.

64. Use of EPO according to any one of claims 42 to 48 for the preparation of medicament for the treatment or use according to any one of claims 54 to 63.
